# EUROPÄISCHE PATENTSCHRIFT

(11) **EP 2 173 392 B1**
(45) Veröffentlichungstag und Bekanntmachung des Hinweises auf die Patenterteilung: **03.08.2011**
(21) Anmeldenummer: 09712787.2
(22) Anmeldetag: 18.02.2009
(51) Int. Cl.: A61L 24/02, C04B 28/34

(54) **ZUBEREITUNG FÜR MAGNESIUMAMMONIUMPHOSPHAT-ZEMENTE**
PREPARATION FOR MAGNESIUM AMMONIUM PHOSPHATE CEMENT
PRÉPARATION POUR CIMENTS DE PHOSPHATE AMMONIACOMAGNÉSIEN

(30) Priorität: 20.02.2008 DE 102008010210
(43) Veröffentlichungstag der Anmeldung: 14.04.2010
(73) Patentinhaber: InnoTERE GmbH, 01307 Dresden (DE)
(72) Erfinder: GBUREK, Uwe, 97080 Würzburg (DE)
(74) Vertreter: Carlsohn, Alexander
(86) Internationale Anmeldenummer: PCT/DE2009/000218
(87) Internationale Veröffentlichungsnummer: WO 2009/103273

(56) Entgegenhaltungen:
- EP-A- 1 296 909
- EP-A- 1 707 543
- US-A1- 2008 020 980
- DRIESSENS, F.C.M; BOLTONG M.G.;WENZ, R.; MEYER J.: "Calcium Phosphates as Fillers in Struvite Cements" KEY ENG. MAT., Bd. 284-286, 2005, Seiten 161-164, XP008108642 Switzerland

## Beschreibung

Die Erfindung betrifft eine Zubereitung für einen Magnesiumammoniumphosphat-Zement, ein Verfahren zur Herstellung eines solchen Zements und Verwendungen des so hergestellten Zementes.

Die Ausheilung von Knochendefekten, deren Ausmaß eine kritische Größe übersteigt, (Critical size defects, CSD) erfordert anwendungsort-spezifisch den Einsatz von Transplantaten oder Implantaten, um ein Einwachsen von Bindegewebe in den Defekt zu verhindern und die verlorengegangene Biofunktionalität wieder herzustellen [1]. In Bezug auf das Einwachsverhalten und die funktionelle Wiederherstellung stellt die autologe Transplantation körpereigenen Knochens einen "goldenen Standard" dar; bei großen Defekten ist sie allerdings mit den Nachteilen der mangelnden Verfügbarkeit und dem Risiko der Zweit-Operation behaftet. Zunehmend erforscht wurden daher in den vergangenen Jahren synthetische Knochenersatzmaterialien. Diese synthetischen Knochersatzwerkstoffe müssen in der klinischen Anwendung verschiedenen Kriterien genügen. Während im kraftbelasteten Bereich vorwiegend mechanische Eigenschaften im Vordergrund stehen und hier vornehmlich hochfeste metallische Werkstoffe, wie Titan, Titanlegierungen, Edelstähle und CoCr-Legierungen, zum Einsatz kommen, wird im nicht- oder nur gering kraft-belasteten Bereich auf ein bindegewebefreies Einwachsen des Werkstoffs und eine gezielte Resorption und Ersatz durch natürlichen Knochen Wert gelegt. Künstliche Werkstoffe mit klinischer Anwendung für solche nicht funktionell kraftbelastete Defekte sind Calciumphosphat-Keramiken [2, 3] bzw. -Zemente [4], Biogläser [2, 5], Kollagene [6, 7] oder lyophilisierte Knochenimplantate. Die Werkstoffe müssen am Anwendungsort nur bedingt mechanische Last aufnehmen und sollen als meist poröse Leitstruktur das Einwachsen von neuem Knochen begünstigen.

Eine zentrale klinische Anforderung an Knochenersatzwerkstoffe ist deren Resorbierbarkeit im physiologischen Milieu unter Neubildung nativer Knochensubstanz. Viele der genannten Werkstoffe können dieser Anforderung nicht oder nur teilweise gerecht werden. Beispielsweise zeigen gesinterte Keramiken aus Hydroxylapatit [8, 9, 10, 11] oder Zemente aus Polymethylmethacrylat [12] keine physiologische Degradierbarkeit. Degradierbare Werkstoffe mit klinischem Einsatz sind etwa Tricalciumphosphat-Keramiken [13] als Pulver oder Granulate, die allerdings keine Stützfunktion im Knochendefekt übernehmen können. Daneben sind in situ formbare und aushärtende mineralische Zemente bekannt, die zum einen zu Hydroxylapatit abbinden können und nur über längere Zeiträume hinweg resorbiert werden oder Zusammensetzungen, die nach dem Abbinden aus Calciumhydrogenphosphat·Dihydrat (Bruschit) bestehen [14] und mittelfristig durch ihre vergleichsweise hohe Löslichkeit in einem Zeitraum von etwa 3 bis 6 Monaten resorbierbar sind [15, 16, 17]. Bedenken gegenüber einer weitreichenden klinischen Anwendung solcher Brushit-Zemente resultieren aus der vergleichsweise geringen mechanischen Festigkeit sowie dem stark sauren pH-Wert der Materialien während und nach dem Abbindevorgang, der zu einer Freiseetzung saurer Phosphationen in das umliegende Gewebe führen kann [18].

Eine weitere und neue Alternative stellen Magnesiumammoniumphosphat-Zemente dar, die innerhalb kurzer Zeit (< 5 bis 10 min) zu Magnesiumammoniumphosphat-Hexahydrat ((NH₄)MgPO₄·6(H₂0), Struvit) abbinden. Struvit stellt eine biologische Calcifizierung dar und wird etwa nach bakterieller Infektion in Nierensteinen gefunden [19]. Zementformulierungen, die als Abbindeprodukt Struvit bilden, wurden verschiedentlich in der Literatur beschrieben [20, 21, 22, 23, 24] und zeigten sehr gute mechanische Eigenschaften mit einer Druckfestigkeit > 50 MPa und einer Abbindezeit von 3 bis 10 min. Die Abbindereaktion der Zemente erfolgt dabei im neutralen pH-Wert-Bereich, da sowohl die Pulverkomponenten (MgHPO₄, Mg₃(PO₄)₂) als auch die flüssige Zementphase ((NH₄)₂HPO₄) nahezu pH-neutral sind. Das Löslichkeitsprodukt von Struvit (LP = 5,21 x 10⁻¹⁵ (pK(sp) = 14,28) bis LP = 2,12 x 10⁻¹³ (pK(sp) =12,67) im pH-Bereich 7,01 - 9,62) [25] läßt eine wesentlich bessere Resorbierbarkeit im Bereich des Knochenersatzes erwarten als etwa auf Hydroxylapatit (pK = 59) oder Tricalciumphosphat (pK = 29) basierende Werkstoffe.

Die literaturbekannten Studien über Struvit-Zemente verwendeten jeweils mehrkomponentige Pulvermischungen aus sekundären (MgHPO₄) und tertiären (Mg₃(PO₄)₂) Magnesiumphosphaten in Verbindung mit Calciumorthophosphaten (α-TCP, β-TCP, HA) als Füllstoffen. Aus DE 100 32 220 A1 sind etwa Zementformulierungen mit einen molaren Mg/P-Verhältnis von 0 < Mg/P < 0,50 und einem Ca/P-Verhältnis von 0 < Ca/P < 1,50 bekannt.

Die Verwendung mehrkomponentiger Pulvermischungen kann hierbei zu Problemen im Hinblick auf die homogene Mischbarkeit bei unterschiedlichen Partikelgrößen oder auf Entmischungsvorgänge bei Lagerung und Transport der Materialien führen. Aufgabe der Erfindung ist es, die Nachteile nach dem Stand der Technik zu beseitigen. Es soll insbesondere eine Zubereitung für die Herstellung von Magnesiumammoniumphosphat-Zementen angegeben werden, die einerseits gute mechanische Eigenschaften und hohe Resorbierbarkeit aufweisen, andererseits aber eine einkomponentige Calcium- und Magnesiumquelle verwenden, so daß eine vereinfachte Zubereitung mit einer definierten Abbindezeit erhalten wird.

Diese Aufgabe wird durch die Merkmale der Ansprüche 1, 17 und 20 gelöst. Zweckmäßige Ausgestaltungen der Erfindungen ergeben sich aus den Merkmalen der Ansprüche 2 bis 16, 18, 19 und 21 bis 25.

Nach Maßgabe der Erfindung ist eine Zubereitung für einen Magnesiumammoniumphosphat-Zement vorgesehen, die
(a) ein Magnesiumcalciumphosphat der Formel MgₓCa_{y}(PO₄)₂O_{z}, in der x + y ≤ 4 ist, x > 1 ist, y > 0 ist, z = x + y - 3 und z ≥ 0 ist
(b) ein Ammoniumsalz; und
(c) Wasser;
   umfaßt, wobei das Ammoniumsalz und das Wasser teilweise oder vollständig als wässerige Lösung des Ammoniumsalzes vorliegen können.

Ist x + y < 3, so ist z zwingend 0. Ist x + y ≥ 3, so liegt z im Bereich von 0 bis 1, wobei vorzugsweise z = 0 ist. In einer besonders bevorzugten Ausführungsform sind x + y = 3 und z = 0.

Das Magnesiumcalciumphosphat hat ein molares Mg/P-Verhältnis im Bereich von 0,5 < Mg/P < 2 und ein molares Ca/P-Verhältnis im Bereich von 0 < Ca/P < 1.

Bevorzugt ist x ≥ 2. Ferner sind ein Magnesiumcalciumphosphat mit 2,0 < x ≤ 2,75 oder Gemische von Magnesiumcalciumphosphaten mit 2,0 < x ≤ 2,75 bevorzugt.

Die Erfindung beruht auf der Erkenntnis, daß ein sukzessiver Ersatz von Magnesiumionen durch Calciumionen bei der Synthese von Verbindungen des Typs MgₓCa_{y}(PO₄)₂O-, die mit wässeriger Ammoniumphosphat-Lösung zu Struvit abbinden, eine definierte Einstellung der Abbindezeit ermöglicht.

Die mittels der erfindungsgemäßen Zubereitung hergestellten ausgehärteten Magnesiumammoniumphosphat-Zemente haben eine hohe Festigkeit, insbesondere eine Druckfestigkeit von mehr als 30 MPa nach 24 h Aushärtung bei 37 °C. Zur Herstellung des Magnesiumammoniumphosphat-Zementes werden die Komponenten der Zubereitung vermischt, bis ein homogenes Gemisch, die Zementpaste, erhalten wird. Während des Abbindevorganges hat die Zementpaste einen neutralen oder nahezu neutralen pH-Wert, was physiologische Anwendungen der Zubereitung erleichtert. Der ausgehärtete Magnesiumammoniumphosphat-Zement ist chemisch im Körperelektrolyten resorbierbar, was auf die vergleichsweise hohe Löslichkeit des Abbindeprodukts Struvit zurückzuführen ist.

Das Magnesiumcalciumphosphat wird vorzugsweise aus einem Gemisch, bestehend aus den Verbindungen Calciumhydrogenphosphat (CaHPO₄), Calciumcarbonat (CaCO₃), Magnesiumhydrogenphosphat Trihydrat (MgHPO₄·3H₂O) und Magnesiumhydroxid (Mg(OH)₂), hergestellt. Alternativ können andere Magnesium- oder CalciumVerbindungen eingesetzt werden, etwa CaO, Ca(OH)₂, CaHPO₄ x 2H₂O, Ca(NO₃)₂, MgO, MgCO₃, Mg(NO₃)₂ etc. Dazu werden pulverisierte Verbindungen in einem vorgegebenen stöchiometrischen Verhältnis vermischt. Das Mischungsverhältnis wird dabei so gewählt, das die Bedingungen für x und y und z in der Formel MgₓCa_{y}(PO₄)₂O_{z} erfüllt sind. Nach dem homogenen Vermischen der Verbindungen wird das so erhaltene Gemisch gesintert, wodurch das Produkt mit der Formel MgₓCa_{y}(PO₄)₂O_{z} erhalten wird. Vorzugsweise wird Sintern bei Temperaturen von über 800 °C und für einen Zeitraum von (1 - 100 h) durchgeführt. Durch das Sintern wird das Magnesiumcalciumphosphat als feste einkomponentige Verbindung erhalten. Der Begriff "einkomponentig" bedeutet hierbei eine homogene Zusammensetzung auch im mikroskopischen Maßstab, es können hierbei aber mehrere, nicht eindeutig identifizierbare kristalline Calcium- und Magnesiumphosphat-Phasen in den Einzelkörnern vorliegen. Anschließend wird der Sinterkuchen zerkleinert und das Zerkleinerungsprodukt auf eine vorgegebene Ausgangspartikelgröße gemahlen. Nach dem Mahlen sollte die Partikelgröße von 0,1 µm bis ca. 100 µm betragen, wobei es vorteilhaft sein kann, Fraktionen mit verschiedenen Partikelgrößen gezielt miteinander zu kombinieren. Besonders bevorzugt liegt die mittlere Partikelgröße des pulverförmigen Magnesiumcalciumphosphat im Bereich von 0,5 bis 10 µm. Durch das Mahlen wird Magnesiumcalciumphosphat in Pulverform erhalten. Das Mahlen führt hierbei nicht nur zur Zerkleinerung der Magnesiumcalciumphosphat-Partikel, sondern auch zu einer partiellen Amorphisierung (mechanische Aktivierung), was zu einer erhöhten Löslichkeit und Reaktivität der durch das Mahlen erhaltenen Magnesiumcalciumphosphat-Partikel führt.

Bevorzugte Magnesiumcalciumphosphate sind z. B. M9_{2,25}Ca_{0,75}(PO₄)₂, Mg_{2,5}Ca_{0,5}(PO₄)₂ und Mg_{2,75}Ca_{0,25}(PO₄)₂. Ebenso können Gemische dieser Verbindungen eingesetzt werden. Da es sich bei den beispielhaft genannten Zusammensetzungen aber nicht um definierte Verbindungen im kristallographischen Sinn handelt, können auch abweichende Zusammensetzungen aus Mg, Ca und (PO₄) gewählt werden. Besonders bevorzugt sind Zusammensetzungen nach der folgenden Formel Mg_{2,0-2,9}Ca_{0,1-1,0}(PO₄)₂

Vorzugsweise umfaßt die Zubereitung das Ammoniumsalz in Form einer wässerigen Lösung. Zur Herstellung des Magnesiumammoniumphosphat-Zementes wird die wässerige Ammoniumsalz-Lösung dann mit dem festen pulverisierten Magnesiumcalciumphosphat bis zum Erhalt eines homogenen Gemisches, vermischt. Das homogene Gemisch wird nachstehend auch als Zementpaste bezeichnet. Das Verhältnis von festem pulverisiertem Magnesiumcalciumphosphat zur wässerigen Ammoniumsalz-Lösung (Pulver/Flüssigkeits-Verhältnis) beträgt vorzugsweise 1,0 bis 5,0 g/ml, stärker bevorzugt 2,5 bis 5,0 g/ml, besonders bevorzugt 2,5 bis 3,5 g/ml.

Alternativ kann das Ammoniumsalz jedoch auch teilweise oder vollständig als festes pulverisiertes Ammoniumsalz mit dem pulverisierten Magnesiumcalciumphosphat vermischt werden. Dieses Vorgehen ermöglicht ein hohes Pulver/Flüssigkeits-Verhältnis, bei dem eine alleinige Lösung des Ammoniumsalzes aufgrund dessen begrenzter Löslichkeit keine ausreichende Konzentration von Ammoniumionen für ein vollständiges Abbinden bereitstellen würde. Das so erhaltene pulverförmige Feststoffgemisch wird anschließend mit Wasser unter Erhalt der Zementpaste vermischt. Das Verhältnis des festen pulverförmigen Gemisches aus Magnesiumcalciumphosphat und Ammoniumsalz zu Wasser (Pulver/Flüssigkeits-Verhältnis) beträgt vorzugsweise 1,0 bis 5,0 g/ml, stärker bevorzugt 2,5 bis 5,0 g/ml, besonders bevorzugt 2,5 bis 3,5 g/ml.

Das Ammoniumsalz ist bevorzugt ein Ammoniumphosphat, besonders bevorzugt ein Diammoniumhydrogenphosphat. Bevorzugt liegt das Diammoniumhydrogenphosphat als eine 3,5 M wässerige Lösung vor. Ebenfalls besonders bevorzugt sind Mischungen aus Diammoniumhydrogenphosphat und Ammoniumdihydrogenphosphat bei vergleichbarer Gesamtkonzentration. Alternative Ammoniumsalze sind ebenfalls verwendbar, beispielsweise insbesondere die die Monoammoniumsalze und/oder die Diammoniumsalze der Citronensäure, Weinsäure, Schwefelsäure und Essigsäure oder deren Gemische.

Die Herstellung des Magnesiumammoniumphosphat-Zementes unter Verwendung der erfindungsgemäßen Zubereitung umfaßt vorzugsweise folgende Schritte:
(a) das Mischen des Magnesiumcalciumphosphats mit dem Ammoniumsalz und dem Wasser und/oder mit einer wässerigen Lösung des Ammoniumsalzes bis zum Erhalt eines homogenen Gemisches (Zementpaste);
(b) Aufbringen oder Einbringen des homogenen Gemisches auf oder in ein Zielobjekt; und
(c) Aushärtenlassen des homogenen Gemisches unter Erhalt des abgebundenen Magnesiumammoniumphosphat-Zementes.

Vorzugsweise wird in Schritt (a) pulverisiertes Magnesiumcalciumphosphat mit einer wässerigen Lösung des Ammoniumsalzes homogen vermischt. Das Ammoniumsalz ist bevorzugt Diammoniumhydrogenphosphat oder eine Mischung aus Diammoniumhydrogenphosphat und Ammoniumdihydrogenphosphat oder Ammoniumdihydrogenphosphat.

Die Abbindezeit der Zementpaste kann durch verschiedene Mechanismen eingestellt werden. Eine Verkürzung der Abbindezeit wird entweder durch eine Verlängerung der Mahldauer und/oder durch Erhöhung der Konzentration des Ammoniumsalzes in der Zubereitung, beispielsweise durch Erhöhung seiner Konzentration in der wässerigen Lösung, erreicht. Alternativ kann (insbesondere bei Erreichen der Löslichkeitsgrenze) ein Teil (oder alles) des Ammoniumsalzes in die Pulverkomponente eingemischt werden.

Die Viskosität der Zementpaste kann durch Verringerung des Pulver/Flüssigkeits-Verhältnisses derart erniedrigt werden, daß die Paste auch durch dünne Kanülen injizierbar ist, ohne daß hierdurch die mechanischen Eigenschaften, wie bei Calciumphosphat-Zementen bekannt, stark erniedrigt werden.

Die erfindungsgemäße Zubereitung wird zur Herstellung eines Magnesiumammoniumphosphat-Zementes verwendet. Der Magnesiumammoniumphosphat-Zement kann für medizinische Zwecke eingesetzt werden, beispielsweise Knochenzement, Knochenersatz und/oder als Knochenfüllstoff oder Knochenklebstoff. Ebenso kann der Magnesiumammoniumphosphat-Zement zur Herstellung von vorgeformten Implantaten verwendet werden. Solche Implantate können beispielsweise durch 3D-Pulverdruck hergestellt werden.

Prinzipiell ist die Kombination von mineralischen Knochenzementen mit verschiedenen Hilfsstoffen aus der Literatur bekannt, insbesondere solchen Hilfsstoffen, die die Injizierbarkeit, Kohäsion, Pastenkonsistenz, Porosität, Resorbierbarkeit, Adhäsion zum Knochen, Lagerstabilität, Kompatibilität mit Wirkstoffen und deren Freisetzung in gewünschter Weise beeinflussen können. Alle diese Hilfsstoffe sind auch mit der hier beschriebenen Zementzusammensetzung kombinierbar.

Vergleichbares gilt für pharmakologische Wirkstoffe. Als Trägermaterial für Wirkstoffe eignet sich der erfindungsgemäße Magnesium-Ammoniumphosphat-Zement in besonderer Weise, weil er aus weitgehend pH-neutralen Komponenten zusammengesetzt ist und auch das Abbindeprodukt neutral reagiert. Besonders bevorzugt ist die Kombination des Zements mit Wirkstoffen, die eine Knochenbildung fördern, Entzündungsreaktionen in der Umgebung des implantierten Materials unterdrücken, Knochenresorption unterdrücken und/oder zur Bekämpfung mikrobieller Infektionen oder Kontaminationen geeignet sind.

Die erfindungsgemäße Zubereitung oder der mittels der erfindungsgemäßen Zubereitung erhaltene Zement kann mit pharmazeutischen Wirkstoffen kombiniert werden. Beispielhafte pharmazeutische Wirkstoffe sind Antibiotika, Knochenwachstumsfaktoren, Zytostatika und Entzündungs-hemmende Wirkstoffe, wobei diese Aufzählung nicht abschließend ist.

Sind die pharmazeutischen Wirkstoffe wasserlöslich, so werden sie vorzugsweise der flüssigen Phase der Zubereitung zugesetzt. Alternativ können die pharmazeutischen Wirkstoffe dem erhaltenen Zement zugesetzt werden. Dabei kann der pharmazeutische Wirkstoff beispielsweise dem pulverförmigen Zement direkt oder verkapselt in einem degradierbaren Polymer zugesetzt werden. Mittels der Verkapselung in einem degradierbaren Polymer kann die Freisetzungskinetik des pharmazeutischen Wirkstoffes vorteilhaft gesteuert werden.

Ferner kann die erfindungsgemäße Zubereitung oder der mittels der erfindungsgemäßen Zubereitung erhaltene Zement mit Hilfsstoffen kombiniert werden. Beispiele von Hilfsstoffen sind Viskositätsbeeinflussende Zusätze zur Verbesserung der Injizierbarkeit sowie der Kohäsion der Zementpaste, etwa wasserlösliche Polymere oder mehrfach geladene Anionen zur elektrostatischen Aufladung der Partikel des Zementes; partikelförmige oder faserartige keramische oder polymere Stoffe zur Verstärkung der Zementmatrix und zur Porenbildung nach Auslaugung, wobei diese Aufzählung nicht abschließend ist.

Der aus der erfindungsgemäßen Zubereitung erhaltene Magnesiumammoniumphosphat-Zement verfügt über eine hohe Druckfestigkeit und eine gute Resorbierbarkeit im menschlichen oder tierischen Körper.

Die Erfindung wird nachstehend anhand von Beispielen näher erläutert.

### Beispiele

Die Herstellung erfindungsgemäßer Magnesiumcalciumphosphate erfolgte durch Sinterung von Pulvermischungen mit der in Tabelle 1 angegebenen Stöchiometrie bei einer Temperatur von 1100 °C und einer Sinterdauer von 5 h. Der Sinterkuchen wurde anschließend manuell auf eine Partikelgröße < 355 µm zerkleinert und in einer Planetenkugelmühle für einen Zeitraum von 10 min bis 24 h vermahlen. Die mittleren Größe der erhaltenen Magnesiumcalciumphosphat-Partikel sind in Tabelle 2 angegeben.

**Tabelle 1**

| Zusammensetzung der Pulvermischungen zur Herstellung der Verbindungen der Formel MgₓCa_{y}(PO₄)₂O_{z} (z = 0) | | | | |
|---|---|---|---|---|
| MgₓCa_{y}(PO₄)₂ | CaHPO₄ [mol] | CaCO₃ [mol] | MgHPO₄ * 3 H₂O [mol] | Mg(OH)₂ [mol] |
| Mg_{1,5}Ca_{1,5}(PO₄)₂ | 1 | 0,5 | 1 | 0,5 |
| Mg_{2,25}Ca_{0,75}(PO₄)₂ | 0,5 | 0,25 | 1,5 | 0,75 |
| Mg_{2,5}Ca_{0,5}(PO₄)₂ | 0,33 | 0,17 | 1,67 | 0,83 |
| Mg_{2,75}Ca_{0,25}(PO₄)₂ | 0,167 | 0,083 | 1,83 | 0,92 |
| *Mg₃(PO₄)₂ | - | - | 2 | 1 |

| | | | | |
|---|---|---|---|---|
| * Vergleichsbeispiel | | | | |

**Tabelle 2**

| Mittlere Partikelgröße in µm von MgₓCa_{y}(PO₄)₂O_{z} (z = 0) | | | |
|---|---|---|---|
| Mahldauer [h] | Mg_{1,5}Ca_{1,5}(PO₄)₂ | Mg_{2,25}Ca_{0,75}(PO₄)₂ | *Mg₃(PO₄)₂ |
| ungemahlen | 41,1±14,2 | 23,8 | 47,8±1,3 |
| 1 h | 16,2±1,6 | 7,7±0,2 | 10,5±2,6 |
| 4 h | 7,3±3,0 | 6,09±0,1 | 7,5±0,4 |
| 24 h | 15,1±1,1 | 0,6±0,1 | 12,7±1,7 |

| | | | |
|---|---|---|---|
| * Vergleichsbeispiel | | | |

Die so erhaltenen Pulver wurden mit einer 3,5 M wässerigen (NH₄)₂HPO₄-Lösung als flüssiger Phase zu einer Zementpaste angemischt. Das Pulver/Flüssigkeits-Verhältnis (P/L) wurde hierbei im Bereich von 1,0 bis 3,5 g/ml variiert. Die Abbindezeiten der Zementpaste wurde anhand des Gilmore-Nadeltests bei 37 °C und > 90 % Luftfeuchtigkeit bestimmt (Tabellen 3 und 4).

**Tabelle 3**

| Abbindezeiten einer Zementpaste aus MgₓCa_{y}(PO₄)₂O_{z} (z = 0)und einer 3,5 M (NH₄)₂HPO₄-Lösung als flüssiger Phase (P/L = 3,0 g/ml), gemessen mittels des Gilmore-Nadeltests | | | |
|---|---|---|---|
| Mahldauer [h] | Mg_{1,5}Ca_{1,5}(PO₄)₂ | Mg_{2,25}Ca_{0,75}(PO₄)₂ | *Mg₃(PO₄)₂ |
| 1 h | 32 min | 14 min | 4 min |

| | | | |
|---|---|---|---|
| * Vergleichsbeispiel | | | |

**Tabelle 4**

| Abbindezeiten einer Zementzusammensetzung aus MgₓCa_{y}(PO₄)₂O_{z} (z = 0)mit einer 3,5 M (NH₄)₂HPO₄-Lösung als flüssiger Phase in Abhängigkeit vom Pulver/Flüssigkeits-Verhältnis (P/L), gemessen mittels des Gilmore-Nadeltests | | | | |
|---|---|---|---|---|
| P/L [g/ml] | Mg_{2,5}Ca_{0,5}(PO₄)₂ | | Mg_{2,75}Ca_{0,25}(PO₄)₂ | |
| | 1 h Mahldauer | 4 h Mahldauer | 1 h Mahldauer | 4 h Mahldauer |
| 3,0 | 8 min | 4 min | 8 min | 3 min 30 sec |
| 2,5 | 13 min | 8 min | 12 min | 6 min 30 sec |
| 2,0 | 18 min | 13 min | 19 min | 14 min |
| 1,5 | 22 min | 19 min | 23 min | 18 min |

Zur Bestimmung der Druckfestigkeit der mittels der erfindungsgemäßen Zubereitung hergestellten Magnesiumammoniumphosphat-Zemente wurde die Zementpaste in Silikonformen gefüllt und 24 h bei 37 °C ausgehärtet. Es resultierten quaderförmige Prüfkörper der Maße 12 x 6 x 6 mm, die mit einer Universalprüfmaschine entlang der Längsachse bis zum Bruch belastet wurden. Aus der Bruchkraft und der Querschnittsfläche wurde die Druckfestigkeit berechnet (Tabellen 5 und 6).

**Tabelle 5**

| Druckfestigkeit von Magnesiumammoniumphosphat-Zementen, her- gestellt aus MgₓCa_{y}(PO₄)₂O_{z} (z = 0) und 3,5 M (NH₄)₂HPO₄-Lösung als flüssiger Phase (P/L = 3,0 g/ml), nach 24 h Aushärtung bei 37 °C und 100 % Luftfeuchtigkeit | | | | | |
|---|---|---|---|---|---|
| Mahldauer¹ [h] | Mg_{1,5}Ca_{1,5}(P O₄)₂ | Mg_{2,25}Ca_{0,75}( PO₄)₂ | Mg₂,₅Ca_{0,5}(P O₄)₂ | Mg_{2,75}Ca_{0,25}( PO₄)₂ | *Mg₃(PO₄)₂ |
| 1 h | 16,0±2,8 | 42,3±9,6 | 21,8±4,0 | 25,7±4,3 | 61,2±6,0 |
| 4 h | 21,2±3,5 | 72,7+8,8 | 77,1±6,4 | 53,8±6,3 | 58,6+9,5 |
| 24 h | - | - | - | - | 42,3±9,6 |

| | | | | | |
|---|---|---|---|---|---|
| ¹ Mahldauer des angegebenen, gesinterten Magnesiumcalciumphosphats * Vergleichsbeispiel - nicht bestimmt | | | | | |

**Tabelle 6**

| Druckfestigkeit von Magnesiumammoniumphosphat-Zementen, hergestellt aus MgₓCa_{y}(PO₄)₂O_{z} (z=0), pulverisiert unter Anwendung verschiedener Mahldauern, und einer 3,5 M (NH₄)₂HPO₄-Lösung als flüssiger Phase mit verschiedenen Pulver/Flüssigkeits-Verhältnissen (P/L), nach 24 h Aushärtung bei 37 °C | | | | |
|---|---|---|---|---|
| P/L [g/ml] | Mg_{2,5}Ca_{0,5}(PO₄)₂ | | Mg_{2,75}Ca_{0,25}(PO₄)₂ | |
| | 1 h Mahldauer | 4 h Mahldauer | 1 h Mahldauer | 4 h Mahldauer |
| 3,0 | 21,8±4,01 | 77,1±6,4 | 25,7±4,3 | 53,8±0,3 |
| 2,5 | 17,4±4,1 | 55,6±7,4 | 20,3±4,3 | 52,7±6,6 |
| 2,0 | 16,7±6,8 | 15,2±4,9 | 15,3±4,9 | 12,2±5,4 |
| 1,5 | 12,4±4,3 | 15,0±4,5 | 13,7±4,5 | 15,0±4,7 |

### Literatur

[1] Aaboe M., Pinholt E.M., Hjortinghansen E.: Healing of experimentally created defects - a review, Brit. J. Oral Maxillofac. Surg. 33 (5): 312-318,1995.
[2] Hench L.L., Anderssan 0.: Bioactive glasses, An introduction to bioceramics, Hench LL, Wilson J (ed.), Singapore: World Scientific, 1993.
[3] Kay J.F.: Bioactive surface coatings for hard tissue biomaterials, CRC Handbook of Bioactive Ceramics 2: 111-122, 1990.
[4] Schmitz J.P., Hollinger JO, Milam S.B.: Reconstruction of bane using calcium phosphate bane cements: a critical review, J. Oral Maxillofac. Surg. 57(9): 1122-1126, 1999.
[5] Hench L.L.: Ceramics, glasses, and composites in medicine, Med. Instrum. 7(2): 136-144, 1973.
[6] Zitzmann N.U., Rateitschak-Pluss E., MarineIla CF: Treatment of angular bane defects with a composite bane grafting material in combination with a collagen membrane, J. Periodont. 74 (5): 687-694, 2003.
[7] Ramshaw JAM., Werkmeister JA, Peters D.E.: Collagen as a biomaterial, Current perspectives on implantable devices 1990,2, Williams D.F. (ed.), Landon: Jai Press Ud., 151-220.
[8] Frayssinet P, Rouquet N, Tourenne F, Fages J, Hardy D, Bonel G. Cell-degradation of calciumphosphate ceramics, Cells and Materials 1993; 3 (4): 383-394.
[9] Gross KA, Berndt CC. Biomedical application of apatites, ReviGws In Mineralogy & Geochemistry 2002; 48: 631-672.
[10] Dorozhin SV, Epple M. Biological and medical significance of calcium phosphates, Angew Chem Int Ed 2002; 41 (17) 3130-3146.
[11] Bohner M. Calcium orthophosphates in medicine: from ceramics to calcium phosphate cements, Injury 2000; 31: S37-!7.
[12] Breusch SJ, Kuhn KD. Bone cements based on polymethylmethacrylate, Orthopade 7003; 32 (1): 41-50.
[13] Bohner M Physical and chemical aspects of calcium phosphates used in spinal surgery, Europ Spine J 2001; 10 S114-S121
[14] Pittet C, Lemaitre J. Mechanical characterization of brushite cements: A Mohr circles' approach, J Biomed Mater Res 2000; 53 (6) 769-780
[15] Theiss F, Apelt D, Brand BA, Kutter A, Zlinszky K, Bohner M, Matter S, Frei C, Auer JA, von Rechenberg B. Biocompatibility and resorption of a brushite calcium phosphate cement, Biomaterials 2005; 26 (21) 4383-4394.
[16] Oberle A, Theiss F, Bohner M, Muller J, Kastner SB, Frei C, Zlinszky K, Wunderlin S, Auer JA, von Rechenberg B. Investigation about the clinical use of brushite-and hydroxylapatite-cement in sheep, Schweizer Archiv für Tierheilkunde 2005; 147 (11) 482-490.
[17] Kuemmerle JM, Oberle A, Oechslin C, Bohner M, Frei C, Boecken I, von Rechenberg B. Assessment of the suitability of a new brushite calcium phosphate cement for cranioplasty - an experimental study in sheep, J Cranio-Maxillofac Surg 2005; 33 (1): 37-44.
[18] Bohner M. pH variations of a solution after injecting brushite cements, Key Engin Mater 2000; 192-1: 813-816.
[19] Coe FL, Evan A, Worcester E. Kidney stone disease, J Clinic Invest 2005; 115 (10): 2598-2608.
[20] Driessens FCM, Boltong MG, Wenz R, Meyer J. Calcium phosphates as fillers in struvite cements, Key Engin Mater 2005; 284-286: 161-164.
[21] Hall DA, Stevens R, El Jazairi B. Effect of water content on the structure and mechanical properties of magnesia-phosphate cement mortar. J Am Ceram Soc 1998; 81(6): 1550-1556.
[22] Sarkar AK. Hydration / dehydration characteristics of struvite and dittmarite pertaining to magnesium ammonium phosphate cement systems. J Mater Sci 1991; 26 2514-2518.
[23] Hipedinger NE, Scian AN, Aglietti EF. Magnesia-ammonium phosphate-bonded cordierite refractory castables: phase evolution on heating and mechanical properties. Cement Concrete Res 2004; 34: 157-164.
[24] Kowalczyk K, Palavit G. Chemical composition of mortars made from the magnesia amidoimidophosphates system. J Mater Sci Letters 1993; 12: 1276-1278.
[25] Sillen LG, Martell AE. Stability constants of metal-ion complexes, 1964, London The Chemical Society.

## Patentansprüche

1. Zubereitung für einen Magnesiumammoniumphosphat-Zement, umfassend
(a) ein Magnesiumcalciumphosphat der Formel MgₓCa_{y}(PO₄)₂O_{z} in der x + y
≤ 4 ist, x > 1 ist, y > 0 ist, z = x + y - 3 und z ≥ 0 ist;
(b) ein Ammoniumsalz; und
(c) Wasser;
wobei das Ammoniumsalz und das Wasser teilweise oder vollständig als wässerige Lösung des Ammoniumsalzes vorliegen können.

2. Zubereitung nach Anspruch 1, **dadurch gekennzeichnet, dass** das Magnesiumcalciumphosphat ein molares Mg/P-Verhältnis im Bereich von 0,5 < Mg/P < 2,0 und ein molares Ca/P-Verhältnis im Bereich von 0 < Ca/P < 1 aufweist.

3. Zubereitung nach Anspruch 1 oder Anspruch 2, **dadurch gekennzeichnet, dass** x ≥ 2 ist.

4. Zubereitung nach einem der vorstehenden Ansprüche, **dadurch gekennzeichnet, dass** das Magnesiumcalciumphosphat ein Magnesiumcalciumphosphat der Formel Mg_{2.0-2.9}Ca_{0.1-1.0}(PO₄)₂ oder ein Gemisch von Magnesiumcalciumphosphaten dieser Formel ist.

5. Zubereitung nach einem der vorstehenden Ansprüche, **dadurch gekennzeichnet, dass** das Magnesiumcalciumphosphat ein gesintertes Magnesiumcalciumphosphat ist.

6. Zubereitung nach einem der vorstehenden Ansprüche, **dadurch gekennzeichnet, dass** das Ammoniumsalz aus der Gruppe ausgewählt ist, die die Monoammoniumsalze und/oder die Diammoniumsalze der Phosphorsäure, Citronensäure, Weinsäure, Schwefelsäure und Essigsäure oder deren Gemische umfasst.

7. Zubereitung nach einem der vorstehenden Ansprüche, **dadurch gekennzeichnet, dass** sie ferner pharmazeutische Wirkstoffe und/oder viskositätsbeeinflussende Hilfsstoffe und/oder porenbildende Hilfsstoffe umfasst.

8. Verfahren zur Herstellung eines Magnesiumammoniumphosphat-Zementes unter Verwendung einer Zubereitung nach einem der Ansprüche 1 bis 7, umfassend
(a) das Mischen des Magnesiumcalciumphosphats mit dem Ammoniumsalz und dem Wasser und/oder mit einer wässerigen Lösung des Ammoniumsalzes bis zum Erhalt eines homogenen Gemisches;
(b) Aufbringen oder Einbringen des homogenen Gemisches auf oder in ein Zielobjekt; und
(c) Aushärtenlassen des homogenen Gemisches unter Erhalt des abgebundenen Magnesiumammoniumphosphat-Zementes.

9. Verfahren nach Anspruch 8, **dadurch gekennzeichnet, dass** Schritt (a) das Mischen des Magnesiumcalciumphosphats mit der wässerigen Lösung des Ammoniumsalzes umfaßt.

10. Verfahren nach Anspruch 8 oder Anspruch 9, **dadurch gekennzeichnet, dass** das Ammoniumsalz in Schritt (a) teilweise oder vollständig als Pulver vorliegt.

11. Verwendung einer Zubereitung nach einem der Ansprüche 1 bis 7 zur Herstellung eines Magnesiumammoniumphosphat-Zementes.

12. Verwendung nach Anspruch 11 zur Herstellung eines Magnesiumammoniumphosphat-Zementes für medizinische Zwecke.

13. Verwendung nach Anspruch 11 oder Anspruch 12 zur Herstellung eines Magnesiumammoniumphosphat-Zementes zum Knochenersatz und/oder als Knochenfüllstoff und/oder Knochenkleber.

14. Verwendung nach einem der Ansprüche 11 bis 13 zur Herstellung eines Magnesiumammoniumphosphat-Zementes für ein vorgefertigtes Implantat.

15. Verwendung nach einem der Ansprüche 11 bis 14, **dadurch gekennzeichnet, dass** die Zubereitung oder der Magnesiumammoniumphosphat-Zement als Träger für pharmazeutische Wirkstoffe fungiert.

## Claims

1. A preparation for a magnesium ammonium phosphate cement comprising
(a) a magnesium calcium phosphate of the formula MgₓCa_{y}(PO₄)₂O_{z}, wherein x + y ≤ 4, x > 1, y > 0, z = x + y - 3 and z ≥ 0;
(b) an ammonium salt; and
(c) water;
wherein the ammonium salt and the water can be present partially or completely as an aqueous solution of the ammonium salt.

2. The preparation according to claim 1 **characterized in that** the magnesium calcium phosphate has a Mg/P molar ratio in the range of 0.5 < Mg/P < 2.0 and a Ca/P molar ratio in the range of 0 < Ca/P < 1.

3. The preparation according to claim 1 or claim 2 **characterized in that** x ≥ 2.

4. The preparation according to any one of the preceding claims **characterized in that** the magnesium calcium phosphate is a magnesium calcium phosphate of the formula Mg_{z.o-2.9}Ca_{0.1-1.0}(PO₄)₂ or a mixture of magnesium calcium phosphates of this formula.

5. The preparation according to any one of the preceding claims **characterized in that** the magnesium calcium phosphate is a sintered magnesium calcium phosphate.

6. The preparation according to any one of the preceding claims **characterized in that** the ammonium salt is selected from the group comprising the monoammonium salts and/or the diammonium salts of phosphoric acid, citric acid, tartaric acid, sulfuric acid and acetic acid or their mixtures.

7. The preparation according to any one of the preceding claims **characterized in that** it further comprises pharmaceutical active ingredients and/or viscosity affecting excipients and/or pore forming excipients.

8. A process for the production of a magnesium ammonium phosphate cement using a preparation according to any one of claims 1 to 7 comprising
(a) mixing the magnesium calcium phosphate with the ammonium salt and the water and/or with an aqueous solution of the ammonium salt until a homogenous mixture is obtained;
(b) application or introduction of the homogenous mixture to or in a target object; and
(c) causing the homogenous mixture to hard obtaining the setted magnesium ammonium phosphate cement.

9. The process according to claim 8 **characterized in that** step (a) comprises mixing of the magnesium calcium phosphate with the aqueous solution of the ammonium salt.

10. The process according to claim 8 or claim 9 **characterized in that** the ammonium salt in step (a) is present partially or completely as powder.

11. The use of a preparation according to any one of claims 1 to 7 for the production of a magnesium ammonium phosphate cement.

12. The use according to claim 11 for the production of a magnesium ammonium phosphate cement for medical purposes.

13. The use according to claim 11 or claim 12 for the production of a magnesium ammonium phosphate cement for bone replacement and/or as bone filler and/or as bone adhesive.

14. The use according to any one of claims 11 to 13 for the production of a magnesium ammonium phosphate cement for a preformed implant.

15. The use according to any one of claims 11 to 14 **characterized in that** the preparation or the magnesium ammonium phosphate cement acts as a support for pharmaceutical active ingredients.

## Revendications

1. Préparation pour un ciment de phosphate d'ammonium magnésium, comprenant
(a) du phosphate de calcium magnésium de formule MgₓCa_{y}(PO₄)₂O_{z} où x + y 4 ≤, x > 1, y > 0, z = x + y - 3 et z ≥ 0 ;
(b) du sel d'ammonium ; et
(c) de l'eau ;
où le sel d'ammonium et l'eau peuvent se présenter dans leur totalité ou en partie sous forme de solution aqueuse de sel d'ammonium.

2. Préparation selon la revendication 1, **caractérisé en ce que** le phosphate de calcium magnésium possède un rapport molaire Mg/P dans la gamme de 0.5 < Mg/P < 2.0 et un rapport molaire Ca/P dans la gamme de 0 < Ca/P < 1.

3. Préparation selon la revendication 1 ou la revendication 2, **caractérisé en ce que** x ≥ 2.

4. Préparation selon l'une quelconque des revendications précédentes, **caractérisé en ce que** le phosphate de calcium magnésium est un phosphate de calcium magnésium de formule Mg_{2.0-2.9}Ca_{0.1-1.0}(PO₄)₂ ou un mélange de phosphates de calcium magnésium de cette formule.

5. Préparation selon l'une quelconque des revendications précédentes, **caractérisé en ce que** le phosphate de calcium magnésium est un phosphate de calcium magnésium fritté.

6. Préparation selon l'une quelconque des revendications précédentes, **caractérisé en ce que** le sel d'ammonium est choisi parmi un groupe composé de sels de monoammonium et/ou de diammonium des acides phosphorique, citrique, tartrique, sulfurique et acétique ou leurs mélanges.

7. Préparation selon l'une quelconque des revendications précédentes, **caractérisé en ce que** laquelle est composée en outre de substances actives pharmaceutiques et/ou d'agents auxiliaires ayant une influence sur la viscosité et/ou d'agents auxiliaires porogènes.

8. Procédé de fabrication d'un ciment de phosphate d'ammonium magnésium en utilisant une préparation selon l'une quelconque des revendications 1 à 7, en
(a) mélangeant le phosphate de calcium magnésium avec le sel d'ammonium et l'eau et/ou avec une solution aqueuse de sel d'ammonium jusqu'à obtention d'un mélange homogène ;
(b) appliquant ou versant le mélange homogène sur ou dans un objet cible ; et
(c) laissant durcir le mélange homogène jusqu'à obtention du ciment de phosphate d'ammonium magnésium durci.

9. Procédé selon la revendication 8, **caractérisé en ce que** l'étape (a) comprend le mélange du phosphate de calcium magnésium avec la solution aqueuse de sel d'ammonium.

10. Procédé selon la revendication 8 ou la revendication 9, **caractérisé en ce que** le sel d'ammonium de l'étape (a) se présente en partie ou dans sa totalité sous forme de poudre.

11. Utilisation d'une préparation selon l'une quelconque des revendications 1 à 7 pour fabriquer un ciment de phosphate d'ammonium magnésium.

12. Utilisation selon la revendication 11 pour fabriquer, à des fins médicales, un ciment de phosphate d'ammonium magnésium.

13. Utilisation selon la revendication 11 ou la revendication 12 pour fabriquer un ciment de phosphate d'ammonium magnésium servant de substitut d'os et/ou de matériel de remplissage des os et/ou de colle à os.

14. Utilisation selon l'une quelconque des revendications 11 à 13 pour fabriquer un ciment de phosphate d'ammonium magnésium pour un implant préfabriqué.

15. Utilisation selon l'une quelconque des revendications 11 à 14, **caractérisé en ce que** la préparation ou le ciment de phosphate d'ammonium magnésium constituent des porteurs de substances actives pharmaceutiques.
